Europäisches Patentamt

European Patent Office

Office européen des brevets

(11) Veröffentlichungsnummer: **0 498 376 A1**

## (12) EUROPÄISCHE PATENTANMELDUNG

(21) Anmeldenummer: **92101835.4**

(22) Anmeldetag: **04.02.92**

(51) Int. Cl.5: **A61M 5/50, A61M 5/32**

(30) Priorität: **04.02.91 ES 9100283**

(43) Veröffentlichungstag der Anmeldung:
**12.08.92 Patentblatt 92/33**

(84) Benannte Vertragsstaaten:
**AT CH DE FR GB IT LI NL SE**

(71) Anmelder: **MEGA-TECH GmbH**
**Rainerstrasse 5**
**A-5310 Mondsee(AT)**

(72) Erfinder: **Redrado, Fernando Melero**
**P. Once de Septiembre 62-64**
**ES-08030 Barcelona(ES)**

(74) Vertreter: **Haft, Berngruber, Czybulka**
**Postfach 14 02 46 Hans-Sachs-Strasse 5**
**W-8000 München 5(DE)**

(54) **Selbstzerstörende Einweg-Sicherheitsspritze.**

(57) Eine Einweg-Sicherheitsspritze, die nach Gebrauch automatisch zerstört wird, weist einen in eine Kammer (2) verschieblichen Kolben (5) auf, der an seiner Stirnseite mit einer Öffnung (10) versehen ist, die mit dem Abschnitt (8) der Kammer (2) hinter dem Kolben (5) kommuniziert und mit einer Membran (14) verschlossen ist. Am Boden (16) der Kammer (2) ist ein spitzes Element (15) vorgesehen, welches bei Bewegung des Kolbens (5) auf den Boden (16) die Membran (14) an der Öffnung (10) des Kolbens (5) durchsticht und damit den Kolben (5) funktionsunfähig macht.

FIG.1

Die Erfindung bezieht sich auf eine selbstzerstörende Einweg-Sicherheitsspritze nach dem Oberbegriff des Anspruchs 1.

Es sind zwar Spritzen bekannt, die für sich beanspruchen, die Sicherheit des Einmalgebrauchs zu bieten, um der möglichen Übertragung von Krankheiten, wie beispielsweise Aids. Hepatitis, usw. vorzubeugen, jedoch sind diese Spritzen entweder sehr kompliziert aufgebaut und somit kostspielig oder es ist erforderlich, daß derjenige, der sie verwendet, nach dem Spritzen einen bestimmten besonderen Handgriff vornimmt, durch den die Spritze unbrauchbar gemacht wird. Unachtsamkeit, Nachlässigkeit oder gar die Absicht, die gleiche Spritze ein zweites Mal zu verwenden, machen den Sicherheitseffekt dieser Spritzen zunichte, so daß sie immer noch als sehr unzuverlässig anzusehen sind.

Aufgabe der Erfindung ist es, eine selbstzerstörende Einweg-Sicherheitsspritze bereitzustellen, mit der ganz sicher ein zweimaliger Gebrauch verhindert ist, so daß eine etwaige Übertragung von ansteckenden Krankheiten oder Infektionen ausgeschlossen wird.

Dies wird erfindungsgemäß mit der im Anspruch 1 gekennzeichneten Einweg-Spritze erreicht. In den Unteransprüchen sind vorteilhafte Ausgestaltungen der Erfindung wiedergegeben.

Bei Verwendung der erfindungsgemäßen Spritze wird der Kolben zerstört, so daß es bei dem Versuch, die Spritze nachzufüllen, nicht möglich ist, irgend eine Flüssigkeit zum zweiten Mal einzusaugen.

Mit der erfindungsgemäßen Spritze wird also ein zweimaliger Gebrauch sicher verhindert, so daß eine Übertragung von ansteckenden Krankheiten unmöglich gemacht wird. D.h., durch die automatische Selbstzerstörung der Spritze am Ende der Verabreichung, ist eine zweimalige Verwendung unmöglich.

Bei der erfindungsgemäßen Spritze ist an dem vorderen Ende der zylindrischen Kammer eine Nadel vorgesehen. Im Innern der Kammer ist ein Kolben verschiebbar geführt, dessen Kolbenstange nach außen verlängert ist. Die Besonderheit der vorliegenden Erfindung besteht nun darin, daß an der Stirnseite des Kolbens eine Membran vorgesehen ist, die durch ein spitzes Element durchbohrt wird, welches am Boden der zylindrischen Kammer angeordnet ist, wenn der Kolben den Boden der zylindrischen Kammer erreicht. Damit ist eine zweimalige Verwendung der Spritze unmöglich. Bei dem spitzen Element kann es sich um ein vorstehendes Teil handeln, das in den Körper der Spritze am Boden der zylindrischen Kammer integriert ist oder um das hintere Ende der Spritzennadel. Im letztgenannten Fall setzt sich die Nadel ins Innere der zylindrischen Kammer fort, wo sie das spitze Ende aufweist, mit dem die Membran am Ende der ersten Verabreichung durchbohrt wird.

Die Membran kann aus einer Kappe bestehen, die zwischen einem an der Stirnseite des Kolbens vorstehenden Teil und einem Ring angeordnet ist. Am Umfang dieses Rings ist ein O-Ring oder eine ähnliche Dichtung angeordnet, um den Kolben gegenüber der Kammerinnenwand abzudichten.

Die Membran kann jedoch auch aus einem scheibenförmigen Körper bestehen, der an der Stirnseite des Kolbens angeklebt oder sonstwie haftend befestigt ist, so daß er ganz oder teilweise die Stirnfläche des Kolbens bedeckt. Hinter der Membran weist der Kolben eine Öffnung auf. Diese kann als Längsöffnung ausgebildet und mit radialen Abzweigungen versehen sein, die in den zweiten Kammerabschnitt münden. Die Kolbenstange kann hohl ausgebildet und mit radialen Öffnungen versehen sein, um den zweiten Kammerabschnitt mit der Umgebung zu verbinden. Das freie Ende der Kolbenstange kann außen einige ring- oder schraubenförmige Verdickungen aufweisen, ähnlich einem Gewinde, und dadurch eine rutschfeste Fläche beim Füllen der Spritze bilden.

Die Nadel kann aus dem Spritzengehäuse herausziehbar oder fest mit dem Spritzengehäuse verbunden sein. Sie ist vorzugsweise durch eine Abdeckung geschützt, wobei die Abdeckung mit dem vorderen Ende des Spritzengehäuse verbunden sein kann.

An dem von der Nadel abgewandten Ende weist das Gehäuse der zylindrischen Kammer einen Einschnitt auf, in den eine Buchse einrastet, die als Kolbenführung und als Herausziehsicherung für den Kolben dient, wodurch eine erneute Verwendung verhindert wird.

Nach einer bevorzugten Ausführungsform der Erfindung weist der Kolben an seiner Stirnseite einen Gewindezapfen auf, an dessen Stirnseite die Membran vorgesehen ist. Die Membran kann dabei durch eine "Abschwächung" der Stirnwand des Gewindezapfens gebildet sein, d.h., der Gewindezapfen und die Membran können einstückig ausgebildet sein.

Die Nadel kann mit einer Buchse verbunden sein, die an ihrem rückwärtigen Teil ein Gewinde zum Ankoppeln an den Gewindezapfen aufweist, der sich von der Kolbenstirnseite koaxial zur Buchse erstreckt. Die Buchse, die die Nadel hält, wird in einem Hals am vorderen Ende des Spritzengehäuses durch ein Gewinde gehalten, das in dem Hals angebracht ist. Wenn durch Drehung der Kolbenstange der Gewindezapfen des Kolbens in der Buchse bis zum Anschlag des Kolbens festgeschraubt worden ist, wird bei fortgesetzter Drehung die Buchse vom Hals der Spritze abgeschraubt.

Der Kolben kann an seiner Rückseite einen Fortsatz mit einem ringförmigen Einschnitt oder einer ringförmigen Erweiterung aufweisen. Ferner kann an der Rückseite des Kolbens bzw. dieses Fortsatzes ein Gewinde angebracht sein, um die an ihrem vorderen Ende ebenfalls mit einem Gewinde versehene Kolbenstange anzuschrauben.

Am hinteren Ende der Spritze ist eine Führungsbuchse für die Kolbenstange angeordnet. Die Führungsbuchse kann einen oder mehrere Vorsprünge zum Festhaken des erwähnten ringförmigen Einschnitts an der Rückseite des Kolbens aufweisen. Damit kann die Kolbenstange nach Verwendung der Spritze und nach Verschrauben des Kolbens mit dem Gewindezapfen in der Buchse sowie dem anschließenden Abschrauben der Buchse von der vorderen Mündung des Spritzengehäuses nach rückwärts bewegt werden, bis der an der Rückseite des Kolbens vorgesehene ringförmige Einschnitt an den Vorsprüngen der Führungsbuchse festgehalten wird, wodurch die Nadel, einschließlich ihrer vorderen Spitze, durch ihre feste Verbindung mit der Buchse dauerhaft im Innern der Kammer der Spritze verbleibt.

In dieser Lage befindet sich der Kolben am hinteren Ende der Kammer, wobei die Kolbenstange durch Abschrauben abmontiert werden kann, wodurch die weggeworfene Spritze weniger Platz einnimmt und ein zufälliger axialer Schub auf die Kolbenstange nicht zum Austritt der Nadel aus dem Spritzengehäuse führt.

Der an der Stirnseite des Kolbens vorgesehen Zapfen und/oder die Ausnehmung der Buchse, in die der Zapfen eingreift, können an der Oberfläche mit einigen schrägen ringförmigen Rillen versehen sein, um den Kolben durch Axialdruck mit der Buchse zu verbinden. Diese ringförmigen schrägen Rillen können also die zuvor genannten entsprechenden Gewinde ersetzen, wodurch die Kopplung zwischen Buchse und Kolben vereinfacht wird.

Zwischen der Buchse und dem vorderen Ende des Spritzenkörpers ist eine scheibenförmige Dichtung angeordnet. Sie schließt nach Durchführung der angegebenen Handgriffe, Drehung und anschließendes Zurückziehen der Kolbenstange aufgrund ihrer elastischen Beschaffenheit automatisch die Öffnung, durch die sich die Nadel erstreckt, bevor sie in die Kammer gezogen wird. Auf diese Weise ist die Kammer nach außen hermetisch abgeschlossen, so daß keine Krankheitserreger oder andere gesundheitsschädliche Substanzen aus dem Inneren der Spritze nach außen gelangen können.

Die Abdeckung oder Kappe, welche mit dem Spritzengehäuse einstückig verbunden sein kann und die Nadel schützt, kann an ihrem vorderen Ende durch einen Stopfen verschlossen sein. Ein solcher Stopfen ist insbesondere dann zweckmäßig, wenn die Spritze für Impfungen verwendet wird, da hierbei im allgemeinen das zu spritzende Mittel bereits in der Spritze angeliefert wird.

An dem hinteren Ende der Nadel, welches zugleich das spitze Element bildet, kann eine seitliche Öffnung vorgesehen sein, die einen zweiten Ausgang für die zu spritzende Flüssigkeit bildet, wobei die Flüssigkeit über diese seitliche Öffnung vorallem dann austritt, wenn das spitze Element die Membran durchbohrt hat.

Nachstehend ist die Erfindung anhand der Zeichnung näher erläutert. Darin zeigen:

Fig. 1    einen Längsschnitt durch eine erste Ausführungsform der Spritze;

Fig. 2    einen Schnitt durch den Fortsatz am Kolben entlang der Linie II-II in Fig. 1;

Fig. 3    einen Längsschnitt durch das vordere Teil der Spritze nach einer anderen Ausführungsform;

Fig. 4    einen Längsschnitt durch eine andere Kolbenvariante;

Fig. 5    einen Längsschnitt durch eine weitere Ausführungsform der Spritze;

Fig. 6    einen Längsschnitt durch den vorderen Teil der Spritze nach Fig. 5 in vergrößerter Wiedergabe und mit teilweise zurückgezogenem Kolben und

Fig. 7    einen Längsschnitt durch die Spritze nach Fig. 5 mit vollständig zurückgezogenem Kolben und abgeschraubter, nur teilweise dargestellter Kolbenstange.

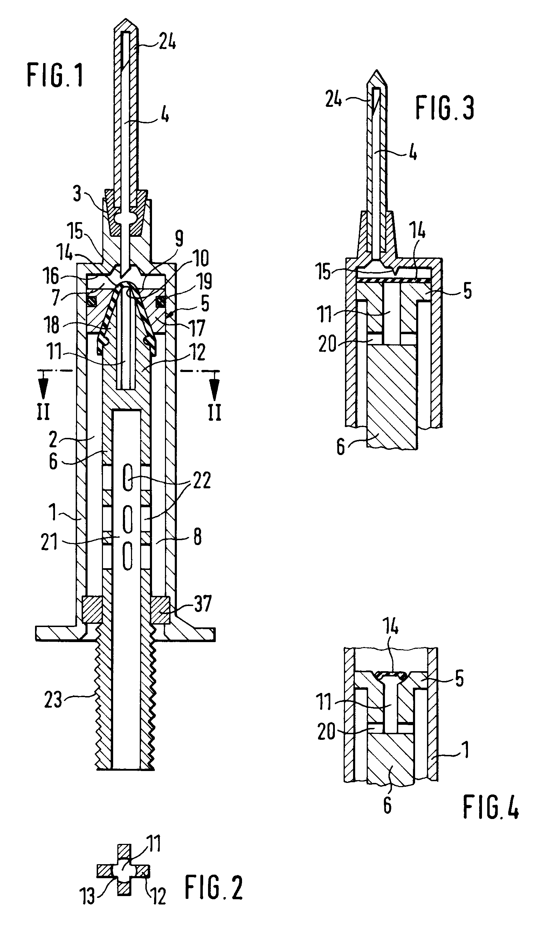

Gemäß Fig. 1 weist die Spritze ein Spritzengehäuse 1 auf, in dem sich eine zylindrische Kammer 2 befindet. Das Spritzengehäuse 1 ist an seinem vorderen Ende mit einer Verjüngung 3 versehen, durch die die Injektionsnadel 4 gesteckt ist, die sich in die Kammer 2 erstreckt.

In der Kammer 2 ist ein Kolben 5 verschiebbar geführt, der an seiner Rückseite mit einer Kolbenstange 6 versehen ist, die sich aus dem hinteren Ende des Spritzengehäuses 1 nach außen erstreckt.

Wenn sich der Kolben 2 zwischen seinen beiden Endstellungen am vorderen bzw. hinteren Ende der Kammer 2 befindet, trennt er die Kammer 2 in einen ersten, vorderen, der Nadel 4 zugewandten Abschnitt 7, in dem sich die Injektionsflüssigkeit befindet, und einen zweiten, hinteren Abschnitt 8, durch den sich die Kolbenstange 6 erstreckt und die keine Flüssigkeit enthält, also mit Luft gefüllt ist.

An seiner der Nadel 4 bzw. dem vorderen Kammerabschnitt zugewandten Stirnseite 9 ist der Kolben 5 mit einer Öffnung 10 versehen, die durch einen sich in Axialrichtung erstreckenden Längskanal 11 gebildet wird, der sich in einen Fortsatz 12 an der Rückseite des Kolbens 5 hinein erstreckt.

Gemäß Fig. 2 ist der Fortsatz 12 im Querschnitt kreuzförmig ausgebildet, wobei der Durchmesser des Längskanals 11 größer ist als die Dikke der Arme des Kreuzes, so daß jeweils seitliche Längsöffnungen 13 in den Ecken zwischen zwei benachbarten Armen gebildet werden, welche mit dem hinteren Abschnitt 8 der Kammer 2, also mit der darin enthaltenen Luft kommunizieren.

Die Öffnung 10 an der Stirnseite 9 des Kolbens ist durch eine Membran 14 verschlossen. Das hintere Ende der Nadel 4, welches in die Kammer 2 bzw. den vorderen Abschnitt 7 hineinragt, ist mit einer Spitze versehen. Dadurch wird ein spitzes Element 15 am vorderen Boden 16 der Kammer 2 gebildet. Das spitze Element 15 liegt dabei in Bewegungsrichtung des Kolbens 2 der Öffnung 10 genau gegenüber, d.h., gemäß Fig. 1 liegen sowohl die Öffnung 10 wie das spitze Element 15 in der Längsmittelachse der Spritze.

Wenn der Kolben 5 außer der in Fig. 1 gezeigten Stellung gegen den Boden 16 der Kammer 2 bewegt wird, durchsticht das spitze hintere Ende 15 der Nadel 4 die Membran 14, so daß die Öffnung 10 freigegeben wird. Damit kommuniziert der vordere Abschnitt 7 mit dem hinteren Abschnitt 8 der Kammer, d.h., der Kolben 2 wird wirkungslos. Die Spritze ist damit unbrauchbar, also ein zweites Mal nicht verwendbar.

Bei der Ausführungsform nach Fig. 1 besteht der Kolben 5 aus einem ersten ringförmigen Abschnitt 17 mit einer sich nach hinten erweiternden Ringöffnung und einem sich von der Kolbenstange 6 in dieRingöffnung des ringförmigen Abschnitts 17 hineinerstreckenden zweiten, sich nach vorne verjüngenden Abschnitt 18, an dem der Fortsatz 12 mit den Längsöffnungen 13 angeordnet ist. Die Membran 14 ist kappenförmig ausgebildet und über den zweiten Abschnitt 18 gestülpt, also zwischen den beiden Abschnitten 17 und 18 eingeklemmt. Am Umfang des Kolbens 5 bzw. des ersten Abschnitts 17 ist in einer Nut ein O-Ring 19 o.dgl. Dichtung angeordnet.

Bei der Ausführungsform nach Fig. 3 ist die Membran 14 als Scheibe ausgebildet, wobei sie auf dem Kolben 2 aufliegt und dessen gesamte Stirnseite 9 bedeckt. Nach Fig. 3 erstrecken sich von dem Längskanal 11 radiale Bohrungen 20 in die Kammer 2 bzw. in den hinteren Kammerabschnitt 8. Bei dieser Ausführungsform kann die Kolbenstange 6 also z.B. zylindrisch ausgebildet und der Längskanal 11 durch eine Längsbohrung in der Kolbenstange 6 gebildet sein.

Weiterhin wird bei der Ausführungsform nach Fig. 3 das spitze Element 15, das die Membran 14 durchsticht, durch einen am Boden 14 der Kammer 2 vorgesehenen Vorsprung gebildet, der der Öffnung 10 gegenüberliegt, also wie diese in der Längsmittelachse der Spritze liegt. Die Injektionsnadel 4 ist bei dieser Ausführungsform exzentrisch angeordnet.

Bei der Ausführungsform nach Fig. 4 ist die Membran 14 in einer Ausnehmung an der Stirnseite 9 des Kolbens 5 befestigt.

Gemäß Fig. 1 weist die Kolbenstange 6, die sich an den Fortsatz 12 anschließt, einen Längskanal 21 auf, der am hinteren Ende der Kolbenstange 6 ins Freie mündet. Die Kolbenstange 6 ist ferner mit radialen Öffnungen 22 versehen, so daß der hintere Abschnitt 8 der Kammer 2 mit der Umgebung kommuniziert. Damit kommuniziert nach dem Durchstechen der Membran 14 der vordere Abschnitt 7 der Kammer 2 ebenfalls mit der Umgebung, so daß die Spritze unbrauchbar ist.

An ihrem hinteren, freien Ende weist die Kolbenstange 6 ring- oder schraubenförmige Verdickungen 28 auf, um eine rutschfeste Fläche zu bilden, die das Füllen der Spritze sicherer macht.

Die Injektionsnadel 4 ist an dem Spritzengehäuse 1 feststehend oder herausschiebbar befestigt. Sie ist durch eine Abdeckung oder Kappe 24 geschützt, die sich bis zum vorderen Ende des Spritzengehäuses 1 erstreckt.

An dem von der Nadel 4 abgewandten Ende weist das Spritzengehäuse 4 an der Innenseite eine Ringnut auf, in der eine Buchse 25 angeordnet ist, welche als Führung und Dichtung für die Kolbenstange 6 sowie als Herausziehsicherung für den Kolben 5 dient. Damit kann der Kolben 5 nicht aus dem Spritzengehäuse 1 gezogen werden, so daß auch das Spritzengehäuse 1 nicht erneut verwendet werden kann, also mit einem neuen Kolben.

Bei der erfindungsgemäßen Spritze schließt der Kolben 5 mit der durch die Membran 14 verschlossenen Öffnung 10 den vorderen Abschnitt 7 vom hinteren Abschnitt 8 der Kammer 2 ab, so daß die Flüssigkeit, die gespritzt werden soll, wie bei einer herkömmlichen Spritze eingesaugt werden kann. Nachdem die Spritze gefüllt ist, wird sie dem Patienten, wie üblich, verabreicht. Wenn der Kolben 5 den Boden 16 der Kammer 2 erreicht, durchsticht das spitze Element 15 die Membran 14 an der Öffnung 10. Damit ist es nicht mehr möglich, die Spritze nachzufüllen. D.h., sie ist unbrauchbar, so daß Risiken vermieden sind, die mit einer zweitmaligen Verwendung der Spritze verbunden sind, wie beispielsweise die Übertragung von Krankheiten, wie Aids, Hepatitis, usw..

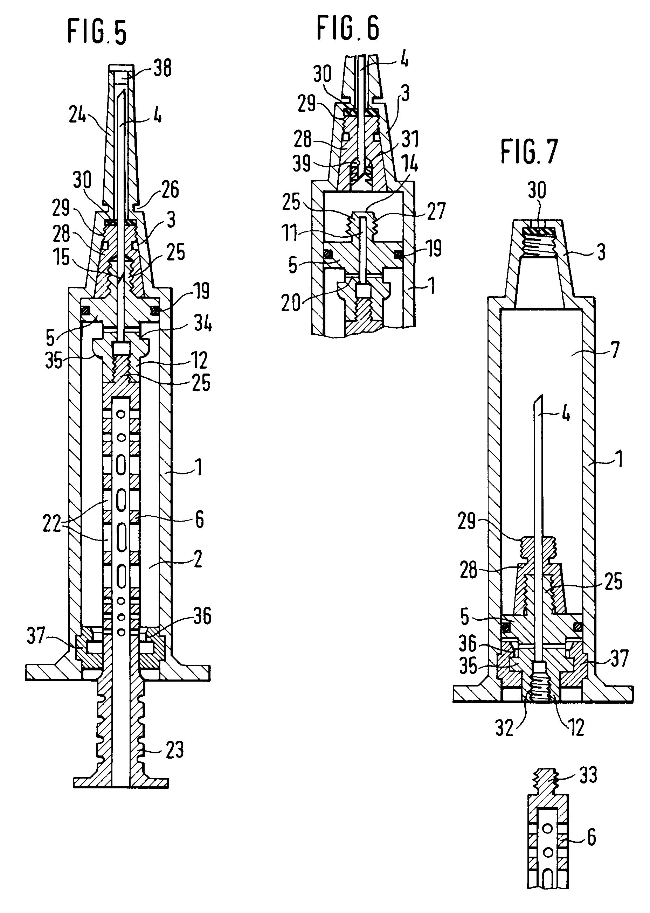

Bei der Ausführungsform nach Fig. 5 bis 7 weist der Kolben 5 an seiner Stirnseite 9 einen Zapfen 25 auf, durch den sich der Längskanal 11 erstreckt. Die Öffnung 10 am vorderen Ende des Längskanals 11 ist dabei wiederum durch eine Membran 11 verschlossen, die jedoch bei dieser Ausführungsform einstückig mit dem Zapfen 25 bzw. dem Kolben 5 ausgebildet ist.

Auch bei dieser Ausführungsform ist die Nadel 4 durch eine hülsenförmige Abdeckung 24 anfänglich geschützt, die jedoch einstückig mit dem Spritzengehäuse 1 ausgebildet ist. Die Abdeckung 24 kann durch Abbrechen an der ringförmigen Abschwächung 26 an ihrer Basis abgezogen werden.

Der Zapfen 25 am Kolben 5 ist mit einem Gewinde 27 versehen und kann damit in eine Buchse 28 geschraubt werden, die in der als Hals ausgebildeten Verjüngung 3 am vorderen Ende des Spritzengehäuses 1 angeordnet ist. Dazu ist die Buchse 28 mit einem Außengewinde 29 versehen, das in ein entsprechendes Innengewinde im Halsabschnitt 3 eingreift. Das Außengewinde 27 an dem Zapfen 25 und das Außengewinde 29 an der Buchse 28 verlaufen in Gegenrichtung; durch Drehung der Kolbenstange 6 und damit des Zapfens 25 ist der Zapfen 25 mit der im Halsabschnitt 3 angeordneten Buchse 28 kuppelbar, die ihrerseits mit dem Halsabschnitt 3 durch Drehung in gleicher Drehrichtung vom Halsabschnitt 3 abgeschraubt wird.

Das Gewinde 27 am Zapfen 25 kann in ein Innengewinde in der Buchse 28 eingreifen. Statt eines Innengewindes kann die Buchse 28 ringförmige Rillen 31 aufweisen, um den Zapfen 25 durch Axialdruck in der Buchse 28 festzuklemmen.

Zwischen der Buchse 28 und dem vorderen Ende des Halsabschnitts 3 des Spritzengehäuses 1 ist eine scheibenförmige Dichtung 30 angebracht.

Um die Gefahr einer Ansteckung durch einen zufälligen Stich oder Kratzer auszuschalten, wird nach Verabreichung der Injektion durch den Verabreichenden die Kolbenstange 6 so gedreht, daß der Zapfen 25 an der Stirnseite 9 des Kolbens 5 in die rückseitige Aufnahme der Buchse 28 mit den Rillen 31 geschraubt wird. Bei Fortsetzung der Drehung wird die Buchse 28 vom Halsabschnitt 3 des Spritzengehäuses 1 abgeschraubt, so daß beim Zurückziehen der Kolbenstange 6 die in der Buchse 28 befestigte Nadel 4 in die Kammer 2 hinein bewegt wird. Wenn die Kolbenstange 6 in ihre zurückgezogene Position bewegt worden ist, greift ferner der ringförmige Vorsprung 36 an der Buchse 37 in die Nut oder Einschnürung 34 an dem rückwärtigen Fortsatz 12 des Kolbens 5 ein, wodurch dieser in dieser Lage festgeklemmt wird.

Dadurch wird die Nadel 4 in der Kammer 2, also im Innern des Spritzengehäuses 1 festgehalten, so daß sie von außen nicht mehr zugänglich ist. Die scheibenförmige Dichtung 30 schließt ihrerseits aufgrund ihrer elastischen Beschaffenheit automatisch die Öffnung, durch die sich zuvor die Nadel 4 erstreckte. Auf diese Weise ist die Kammer 2 mit der darin angeordneten Nadel 4 hermetisch gegenüber der Umgebung abgeschlossen.

Wenn die Kupplung des Kolbens 5 mit der Buchse 28 durch die schrägen ringförmigen Rillen 31 erfolgt, tritt die Kupplung automatisch am Ende der Verabreichung der Spritze ein, d.h., wenn der Zapfen 25 die Buchse 28 erreicht. Nachdem die Nadel 4, wie vorstehend angegeben, durch Zurückziehen der Kolbenstange 6 in das Spritzengehäuse hinein bewegt worden ist, kann die Kolbenstange 6 durch Aufschrauben der Gewinde 32 und 33 von dem Kolben 6 abgeschraubt werden. Damit benötigt die weggeworfene Spritze weniger Platz. Darüber hinaus kann kein ungewollter Axialschub erfolgen, durch den im Extremfall die Nadel 4 wieder herausbefördert werden würde. Ebenfalls aus Gründen der Sicherheit und zur Verringerung des Platzbedarfes erfolgt der Verkauf der Spritze vorteilhafterweise mit abgeschraubter Kolbenstange 6.

Wenn die Spritze für Impfungen verwendet werden soll - in diesem Fall ist es üblich, daß das Mittel, das gespritzt werden soll, ursprünglich in der Spritze enthalten ist - ist an dem Ende der Abdeckung 24 ein Stopfen 38 befestigt, um zu verhindern, daß das Impfmittel durch die Nadel 4 austritt.

Die Nadel 4 weist am rückseitigen Ende, an der sich das spitze Element 15 befindet, eine seitliche Öffnung 39 auf, die einen zweiten Ausgang für die zu spritzende Flüssigkeit bildet, und insbesondere dann erforderlich ist, wenn das spitze Element 15 in die Membran 14 eingedrungen ist, um den Rest der Flüssigkeit zu verabreichen. D.h., die Öffnung 39 gewährleistet den Flüssigkeitsaustritt bis zum Ende der Verabreichung. insbesondere dann erforderlich ist, wenn das spitze Element 15 in die Membran 14 eingedrungen ist, um den Rest der Flüssigkeit zu verabreichen. D.h., die Öffnung 39 gewährleistet den Flüssigkeitsaustritt bis zum Ende der Verabreichung.

**Patentansprüche**

1. Selbstzerstörende Einweg-Sicherheitsspritze mit einer Nadel (4) an einem Ende und einer Kammer (2), in der ein Kolben (5) mit einer Kolbenstange (6) verschiebbar angeordnet ist, der die Kammer 2 in einen ersten, der Nadel (4) zugewandten Abschnitt (7) und in einen zweiten Abschnitt (8), durch den sich die Kolbenstange (6) erstreckt, trennt, *dadurch gekennzeichnet,* daß der Kolben (5) an seiner, dem ersten Kammerabschnitt (7) zugewandten Stirnseite (9) eine mit dem zweiten Kammerabschnitt (8) kommunizierende Öffnung (10) aufweist, die durch eine Membran (14) verschlossen ist, und an dem Boden (16) der Kammer (2) ein spitzes Element (15) vorgesehen ist, welches bei Bewegung des Kolbens (5) auf den Boden (16) der Kammer (2) die Membran (14) an der Öffnung (10) des Kolbens (5) durchsticht.

2. Spritze nach Anspruch 1, *dadurch gekennzeichnet,* daß das spitze Element (15) durch einen Vorsprung am Boden (16) der Kammer (2) gebildet wird.

3. Spritze nach Anspruch 1, *dadurch gekennzeichnet,* daß das spitze Element (15) durch ein spitz ausgebildetes, rückseitiges Ende der Nadel (4) gebildet wird, welches über den Boden (16) in die Kammer (2) hineinragt.

4. Spritze nach einem der vorstehenden Ansprüche, *dadurch gekennzeichnet,* daß die Membran (14) aus einer Scheibe besteht, die an der Stirnseite (9) des Kolbens (5) angeordnet ist.

5. Spritze nach einem der Ansprüche 1 bis 3, *dadurch gekennzeichnet,* daß der Kolben (5) aus einem ersten, ringförmigen Abschnitt (17) und einem sich von der Kolbenstange (6) in den ersten Abschnitt (17) erstreckenden zweiten Abschnitt (18) mit der mit dem zweiten Kammerabschnitt (46) kommunizierenden Öffnung (10) besteht, und die Membran (14) als den zweiten Abschnitt (18) abdeckende Kappe ausgebildet ist.

6. Spritze nach einem der vorstehenden Ansprüche, *dadurch gekennzeichnet,* daß die Membran (14) mit dem Kolben(5) einstückig ausgebildet ist.

7. Spritze nach einem der vorstehenden Ansprüche, *dadurch gekennzeichnet,* daß der Kolben (5) an seiner Stirnseite (9) einen Zapfen (25) mit der durch die Membran (14) verschlossenen Öffnung (10) aufweist, wobei der Zapfen (25) mit einer in einem Halsabschnitt (3) des Spritzengehäuses (1) angeordneten, von der Nadel (4) durchragten Buchse (28) kuppelbar ist, die ihrerseits mit dem Halsabschnitt (3) kuppelbar ist.

8. Spritze nach Anspruch 7, *dadurch gekennzeichnet,* daß der Zapfen (25) zur Kupplung mit der Buchse (28) und/oder die Buchse (28) zur Kupplung mit dem Halsabschnitt (3) mit einem Gewinde (27,29) versehen ist.

9. Spritze nach Anspruch 8, *dadurch gekennzeichnet,* daß die Buchse (28) und/oder der Halsabschnitt (3) mit einem Gegengewinde oder mit ringförmigen Rillen (31) zum Festklemmen des Zapfens (18) bzw. der Buchse (19) durch Axialdruck versehen ist.

10. Spritze nach einem der Ansprüche 7 bis 9, *dadurch gekennzeichnet,* daß an dem Boden des Halsabschnitts (3) eine Dichtung (30) vorgesehen ist.

11. Spritze nach einem der vorstehenden Ansprüche, *dadurch gekennzeichnet,* daß an dem rückseitigen Endabschnitt der Nadel (4) eine seitliche Öffnung (39) als zweiter Ausgang für die zu spritzende Flüssigkeit vorgesehen ist.

12. Spritze nach einem der vorstehenden Ansprüche, *dadurch gekennzeichnet,* daß die Kolbenstange (6) einen nach außen mündenden Längskanal (21) aufweist.

13. Spritze nach einem der vorstehenden Ansprüche, *dadurch gekennzeichnet,* daß die von der Membran (14) verschlossene Öffnung (10) an der Stirnseite (9) des Kolbens (5) über einen Längskanal (11) mit dem zweiten Kammerabschnitt (8) und/oder mit dem Längskanal (21) in der Kolbenstange (6) kommuniziert.

14. Spritze nach Anspruch 13, *dadurch gekennzeichnet,* daß der zweite Kammerabschnitt (8) mit dem Längskanal (21) in der Kolbenstange (6) kommuniziert

15. Spritze nach einem der vorstehenden Ansprüche, *dadurch gekennzeichnet,* daß die Nadel (4) durch eine mit dem Spritzengehäuse (1) verbundene Abdeckung (24) geschützt ist.

16. Spritze nach Anspruch 14, *dadurch gekennzeichnet,* daß die Abdeckung (24) einstückig mit dem Spritzengehäuse (1) ausgebildet ist und eine Abschwächung (26) an ihrer Basis aufweist.

17. Spritze nach einem der vorstehenden Ansprüche, *dadurch gekennzeichnet,* daß an dem von der Nadel (4) abgewandten Ende der Kammer (2) eine Buchse (37) als Führung und Dichtung für die Kolbenstange (6) und als Herausziehsicherung für den Kolben (5) befestigt ist.

18. Spritze nach Anspruch 17, *dadurch gekennzeichnet,* daß an der Rückseite des Kolbens (5) ein Fortsatz (12) mit einer Erweiterung (35) vorgesehen ist und die Buchse (37) an der dem Kolben (5) zugewandten Seite wenigstens einen radial nach innen gerichteten elastischen Vorsprung (36) aufweist, der bei zurückgezogenem Kolben (5) die Erweiterung (35) übergreift.

19. Spritze nach einem der vorstehenden Ansprüche, *dadurch gekennzeichnet,* daß der Kolben (5) und die Kolbenstange (6) lösbar verbunden sind.

FIG.1

FIG.3

FIG.2

FIG.4

EP 0 498 376 A1

FIG. 5

FIG. 6

FIG. 7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-8 909 075 (DAVSA SEVENTY-FIFTH PTY LTD) | 1-6, 11-14 | A61M5/50 A61M5/32 |
| Y | * Seite 6, Zeile 25 - Seite 7, Zeile 36; Abbildungen * | 7-10, 15-19 | |
| | --- | | |
| Y | US-A-4 888 002 (BRAGINETZ ET AL) | 7-10, 15-19 | |
| | * das ganze Dokument * | | |
| | --- | | |
| X | NL-A-8 801 072 (RUTTEN) | 1-4,6, 11,17-19 | |
| A | * Abbildungen * | 7-10 | |
| | --- | | |
| X | US-A-4 687 467 (CYGIELSKI) | 1-3,6, 12-14 | |
| Y | * Spalte 2, Zeile 39 - Spalte 3, Zeile 2; Abbildungen * | 4,5,15, 16 | |
| | --- | | |
| P,Y | US-A-4 994 034 (BOTICH ET AL) | 4,5,15, 16 | |
| A | * Zusammenfassung; Abbildungen * | 1-3,7, 10,15,16 | RECHERCHIERTE SACHGEBIETE (Int. Cl.5 ) |
| | --- | | |
| X | FR-A-2 298 340 (BLANIE) | 1,2,4,6, 12,13 | A61M |
| Y | * das ganze Dokument * | 7-11 | |
| | --- | | |
| Y | EP-A-0 347 742 (VENTURINI) * das ganze Dokument * | 7-11 | |
| | --- | | |
| X | WO-A-9 011 790 (MCMAHON) | 1-3,5, 12-14,17 | |
| A | * Seite 16, Zeile 12 - Seite 19, Zeile 8; Abbildungen 11-23 * | 15,16, 18,19 | |
| | --- | | |
| X | FR-A-2 606 643 (CONGIO ET AL) | 1,2,5, 12-14,17 | |
| | * das ganze Dokument * | | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 14 MAI 1992 | CLARKSON P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EP 92 10 1835
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| | | | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 14 MAI 1992 | CLARKSON P. |